# EUROPEAN PATENT APPLICATION

(11) **EP 0 938 897 A1**
(43) Date of publication of application: **01.09.1999**
(21) Application number: 98310634.5
(22) Date of filing: 23.12.1998
(51) Int. Cl.: A61K 31/35

(54) **Telomerase inhibitor**

(30) Priority: 26.12.1997 JP 36128297
(71) Applicant: JAPANESE FOUNDATION FOR CANCER RESEARCH, Tokyo (JP); Tsuruo; Takashi, Setagaya-ku, Tokyo (JP); Ito En, Ltd., Shibuya-ku, Tokyo (JP)
(72) Inventor: Tsuruo, Takashi, Setagaya-ku, Tokyo (JP); Imad, Naasani, Toshima-ku, Tokyo (JP); Seimiya, Hiroyuki, Adachi-ku, Tokyo (JP); Sugano, Haruo, Suginami-ku, Tokyo (JP)
(74) Representative: Schlich, George William

(57) **Abstract**

Catechins, known to be components of tea, are used to inhibit the enzymic activity of telomerase, an enzyme that synthesizes the telomeric DNA which is responsible for stabilizing chromosomes during cell growth. This telomerase inhibiting activity is also useful in cancer prevention and treatment.

## Description

### Background of the Invention

### 1. Field of the Invention

The present invention relates to a telomerase inhibitor which uses catechins as an effective component and is useful as a cancer prevention agent, anticancer agent, agent to delay cancer progression, or the like.

### 2. Description of the Related Art

Various surgical, internal and, radiological antineoplastic procedures, and combinations thereof have been developed for the treatment of cancer. Extensive investigations are also being done to prevent cancer or on prophylactic means to regard the progression of a cancer as much as possible so as to prevent its transition to the fatal stage. Yet in all cases, it is evident that a further understanding as to the mechanism of the genesis and progression of cancers is essential in order to develop effective methods for their prevention and treatment.

Recently, the presence of an enzyme called telomerase has drawn attention as a new target in cancer therapy. This telomerase, a reverse transcriptase, synthesizes the telomeric DNA (hereinafter referred to as telomere) which is involved in the stabilization of a chromosome during cell growth. This telomere is a segment composed of a linear DNA at the end of the chromosome, and the presence of the telomere stabilizes the chromosome itself and prevents aberrations caused by binding between chromosomes.

Research has revealed that telomeres in human tissues are shortened with age, and that cells die as this shortening progresses; thus the involvement of telomeres with the aging process and cell division cycle has become clear. Furthermore, it has been reported that telomeres in cancer cells are shorter than those in surrounding normal cells, which has further drawn attention to the telomere as an interesting target in cancer research.

Moreover, it has been reported that activity of telomerase, a telomere-synthesizing enzyme, was detected in cells of 85% of all cancers (for example, Kim, N.W. et al., Science, 266, 2011-2015, 1994; Healy, K.C., Oncol. Res., 7, 121-130, 1995; Raymond, E. et al., Biotechnology, 7, 583-591, 1996). In contrast, almost all normal somatic cells are known to be telomerase negative. On the basis of these observations, telomere and telomerase have drawn further attention as potential targets in cancer treatment.

Meanwhile, effect of tea in the prevention and control of cancer is being studied. Yang et al. (Yang, C.S. and Wang, Z-Y., J. Natl. Cancer Inst., 85, 1038-1049, 1993) and Fujiki et al. (Fujiki, H. et al., Nutrition Reviews, 54, 67-70, 1996) discussed the anticancer activity of green tea from the epidemiological point of view in their review articles. Furthermore, Liao et al. (Liao, S. et al., Cancer Letter, 96, 239-243, 1995) reported that epigallocatechin gallate (EGCG) contained in tea effectively suppressed cancer cell growth in nude mice with experimentally-induced tumors. Further, Taniguchi et al. (Taniguchi, S. et al., Cancer Letters, 65, 51-54, 1992) reported that oral administration of EGCG suppressed metastasis of malignant melanoma cells. However, none of these reports described the mechanisms underlying these effects of green tea or EGCG on cancer. In particular, these reports did not mention the effects of green tea or EGCG on telomerase, or the mechanism of anticancer activity associated with telomerase.

Although the utility of a telomerase inhibitor in cancer treatment could be surmised based on the hypothesis that the inhibition of telomerase activity in a cancer cell renders its chromosome less stable thereby shortening the life span of the cancer cell; However, there are no reports to date regarding a substance which has an effective telomerase inhibiting activity and properties satisfactory for pharmaceutical use. Nor is there a reported case in the life span of cancer cell was effectively shortened by the use of a telomerase inhibitor.

### Summary of the Invention

The object of the present invention is to provide a telomerase inhibitor having telomerase inhibiting activity, which is useful in providing an understanding of not only the involvement of telomere and telomerase with cancer, but also its prevention and treatment, whereby the telomerase inhibitor has the safe and appropriate properties for pharmaceutical use.

A telomerase inhibitor of the present invention is characterized in using or containing catechins as an effective component. A telomerase inhibitor of the present invention can be used as a reagent for use in various experiments on telomerase inhibition per se or those involving telomerase inhibition, and further as a cancer prevention agent, anticancer agent, or the like by preparing it in a pharmaceutical preparation. Further, by using a telomerase inhibitor of the present invention, the life span of cancer cells can be shortened to kill the cells, or the progression of cancer can be effectively controlled by arresting its growth and malignancy.

Catechins used in the present invention have unconventionally strong telomerase inhibiting activity, and can provide a telomerase inhibitor which is useful in providing an understanding of the involvement of telomerase with a cancer, and its prevention and treatment, and which possesses the safe and appropriate properties for pharmaceutical use.

### Brief Description of the Drawings

Figure 1 shows the telomerase activity of different compounds.
Figure 2 shows changes in telomerase activity as a function of EGCG concentration.
Figure 3 shows a Dixon plot for the telomerase inhibiting activity of EGCG.
Figure 4 shows the telomerase inhibiting activity of EGCG in a cell.
Figure 5 shows the effect of EGCG on cell growth.
Figure 6 shows the effect of EGCG on cell growth.
Figure 7 shows the distribution of TRF lengths from HT29 cells at PD67 to PD70 in subcultures in the presence or absence of EGCG (15 µM).

### Detailed Description of the Invention and Preferred Embodiments

Commercially available catechins, or catechins extracted and isolated from tea by a known method can be used as an effective component of a telomerase inhibitor of the present invention.

Catechins from tea can be extracted and isolated, for example, by extracting tea leaves with hot water or a hydrophilic organic solvent, removing caffein from the extract by extraction with chloroform, then further extracting with an organic solvent such as ethyl acetate to obtain a tea-catechin mixture, and finally isolating the catechins from this mixture by column chromatography using a hydrophilic organic solvent such as acetone as the eluant.

For example, extracts extracted from green tea, which contain catechins, or crude or purified products prepared from such extracts can be used. An example of a green tea extract is THEA-FLAN 90S (product name, ITO EN, Ltd).

Such catechins from tea can be isolated, for example, by methods described in Japanese Patent Laid-open (Kokai) No. 182479/92, Japanese Patent Laid-open (Kokai) No. 182480/92, Japanese Patent Laid-open (Kokai) No. 9607/94, Japanese Patent Laid-open (Kokai) No. 70105/75, and Japanese Patent No. 2703241.

Further, a green tea extract prepared as follows can also be used. First, leaves and stems of green tea plant are extracted with hot water to fractionate an extract, then this extract is concentrated while separating the solids, if necessary. Concentration can be appropriately done under vacuum. The concentrate thus obtained is sterilized by heating, if necessary, then dried for use as a catechin-containing extract. Spray drying is appropriately used. Undried material can also be used, but the dried powder is preferable because of its ease-of-handling and improved preservability.

A green tea extract containing at least 35% by weight, preferably at least 90% by weight tea polyphenols and at least 10% by weight, preferably at least 40% by weight (-) epigallocatechin gallate can appropriately be used. Further, the caffein content in the extract is preferably 10% or less than 10% by weight. The catechin content in the extract can be increased with the level of purification, which can be chosen as a function of the intended use. Catechins in the extract can be purified by an appropriate combination of a conventional extracting process, isolation process, column chromatography, and the like.

Examples of catechins to be used in the present invention include (-) epigallocatechin gallate (EGCG), (-) epigallocatechin (EGC), (-) epicatechin gallate (ECG), (-) epicatechin (EC) and (+)catechin. Derivatives of these catechins can also be used to the extent that efficacy of the present invention can be attained. Two or more of these catechins in combination can also be used. Of these catechins, epigallocatechin and epicatechin gallate are preferable, and epigallocatechin gallate is particularly preferable.

A telomerase inhibitor of the present invention can be prepared by incorporating a catechin as an effective component into an appropriate solvent, if necessary. Commercial catechins or highly-purified catechins obtained by extraction from tea can best be used by incorporating the catechin into an oil-in-water (O/W) emulsion, prepared, for example by mixing the catechin with a vegetable oil, emulsifying agent (e.g., Tween or Emulgene), and ascorbic acid (antioxidant), then lyophilizing the resulting emulsion. This emulsion is expected to permit a better intestinal absorption of catechins, thus a more potent antitelomerase effect. An example of the composition before lyophilization is 5% by weight vegetable oil, emulsifying agent, or the like, 0.1% by weight antioxidant, and 5% by weight catechin. A preferable catechin content in the inhibitor after lyophilization is preferably 90-95% by weight. The inhibitor can be diluted for use, if necessary.

Further, when the abovementioned tea extract is used, the extract itself or the extract with an addition of an appropriate solvent can be used to obtain a telomerase inhibitor of the present invention. If an extract is used, the dried powder is preferable.

A pharmaceutical composition which contains a catechin having telomerase inhibiting activity as an active component can appropriately be used as a cancer prevention agent or carcinostatic agent. The carcinostatic effect includes the anticancer effect by which cancer cells are directly attacked, the effect by which the progression of cancer (malignancy) is retarded, or the like. A pharmaceutical composition having a catechin as an effective component can be formulated into various forms suited for the purpose of the treatment, by a conventional method using pharmaceutically acceptable carriers, diluents, or the like. Examples of such forms of pharmaceutical compositions include solid formulations such as tablets, pills, dispersible powders, granules, capsules, and suppositories, liquid formulations such as injections, suspensions, syrups, and emulsions, and semi-solid formulations such as plasters.

The amount of a catechin used in a pharmaceutical composition as an effective component can be, for example, 100 to 500 mg (approximately 90 to 95% by weight of the composition) for a tablet or capsule if a commercially available catechin or a purified preparation is used. A tea extract can be used without further purification in certain formulations, or it can be formulated into various forms suited to the intended treatment, by a conventional method using pharmaceutically acceptable carriers, diluents, or the like in addition to the extract.

The dosage of a catechin used in a pharmaceutical composition can conveniently be determined as a function of the intended treatment, the nature of the condition being treated, or the like. For example, the dosage for adults can be in the range of about 500 to 2000 mg per day as the amount of effective chemical component, but it is not so restricted. Furthermore, the abovementioned lyophilized O/W emulsion can appropriately be used as a formulation or as a material for formulations.

Furthermore, various additives conventionally used in formulations can be incorporated in the pharmaceutical formulation of the present invention within a range so as not to inhibit the desired action of the catechin. Moreover, a telomerase inhibitor of the present invention could be rendered even more effective in the treatment of cancer by the co-administration of a chemotherapeutic agent (anticancer agent) to the cancer cells being attacked by the telomerase inhibitor.

A catechin, the effective component of the telomerase inhibitor of the present invention, can be obtained from tea components, is very safe, shows an effective activity in a small amount, and is very appropriate for use as a drug, particularly for the prevention or treatment of cancers in which telomerase is expressed.

The present invention will be further illustrated by the following examples. Unless otherwise stated, "%" means "% by weight."

### Production Example 1

Green tea leaves (20 g) were immersed in 300 ml of hot water (60°C) for 15 minutes for extraction. The extract was then filtered and the filtrate was concentrated at 40°C under vacuum using a rotary evaporator. After concentrating to about 40 ml, the resulting fluid was centrifuged (3,000 rpm, 10 minutes), and the supernatant thus obtained was lyophilized to obtain a pale brown dried powder (4.7 g) (powder sample 1).

Results of analysis as to the content of polyphenol, catechins, and other components contained in the dried powder thus obtained were as follows:

| | |
|---|---|
| Tea polyphenols | 41.5% by weight |
| (-)-Epigallocatechin gallate | 15.1% by weight |
| (-)-Epigallocatechin | 6.6% by weight |
| (-)-Epicatechin gallate | 3.5% by weight |
| (-)-Epicatechin | 2.3% by weight |
| Caffein | 7.3% by weight |

### Example 1

### Measurement of telomerase inhibiting activity in cell-free system

Telomerase inhibiting activity of EGCG, EGC, ECG, and EC (all products of Sigma) was measured according to the Telomeric Repeat Amplification Protocol (TRAP) method described by Kim et al. (Kim, N.W. et al., Science, 266, 2011-2015, 1994) as follows:

### (1) Preparation of cell extract

Cells were washed with ice-cold PBS (phosphate buffered saline) and suspended again in PBS to count the cells. After centrifuging this cell suspension, the resulting cell mass was suspended in a TRAP assay buffer (10 mM Tris-HCl, pH 7.5, 1 mM MgCl₂, 1 mM EDTA, 0.5% CHAPS, 10% glycerine, 5 mM 2-mercaptoethanol, and 0.1 mM 4-(2-aminoethyl)-benzenesulfonyl fluoride hydrochlorine), then the suspension was quick-frozen with liquid nitrogen. After melting, the suspension was allowed to stand in an ice bath for 30 minutes for extraction, then the resulting extract was centrifuged to obtain the supernatant as a cell extract for activity measurement.

### (2) Reaction and treatment after reaction

A test compound, TS primer (50 ng), 50 µM dNTPs, and T4g32 protein (Behringer-Manheim) were added to a specified volume of the cell extract (corresponding to 200 to 1000 cells), and the admixture was incubated at 20°C for 30 minutes. After reaction, CX primer (100 ng) and an internal standard for PCR, ITAS (10⁻¹⁸ g), were added to the reaction solution, then PCR was performed in the presence of AmpliTaq DNA polymerase (2 U), i.e., (94°C, 40 seconds → 50°C, 40 seconds → 72°C, 60 seconds) x 30 cycles → 72°C, 2 minutes, to obtain PCR products.

Base sequences of the primers used in the abovementioned procedure are as follows:

### TS primer:

5'-AATCCGTCGAGCAGAGTT-3' (SEQ ID NO: 1)

### CX primer:

5'-GTGCCCTTAACCCTTACCCTTACCCTAA-3' (SEQ ID NO: 2)

### ITAS:

5'AATCCGTCGAGCAGAGTTGTGAATGAGGCCTTCGAGGCTCTGAAG
AGAAGCACCCTGCTCAACCCCAACCAGCGGCTGCCTAAGGTGGAGATCCT
GCGCAGTGCCATCCAGTACATTGAGCGCCTATTAGGGTAAGGGTAAGGGT
AAGGG-3' (SEQ ID NO: 3)

In the in vitro reaction system, telomerase performs addition synthesis of telomere at the 3' end of the TS primer. Of the 24 bases at the 3'-end side of the CX primer, 21 bases are identical to a telomere sequence, hence the telomere product can be amplified by PCR with a combination of TS primer and CX primer. The 5' end pail and the 3' end part of the ITAS sequence are made identical to the TS primer sequence and the CX primer sequence, respectively.

### (3) Detection and quantitative analysis of reaction products

The PCR products obtained in the abovementioned procedure were resolved by polyacrylamide gel electrophoresis and the resulting DNA bands stained with SYBR Green (Takara Shuzo) were detected by irradiation with a UV transilluminator. The detected bands were photographed. The intensity of each band was quantitatively analyzed using the NIH Image 1.60 software program. Telomerase activity was expressed as the intensity of a sample band relative to that of the bad corresponding to the ITAS fragment (control).

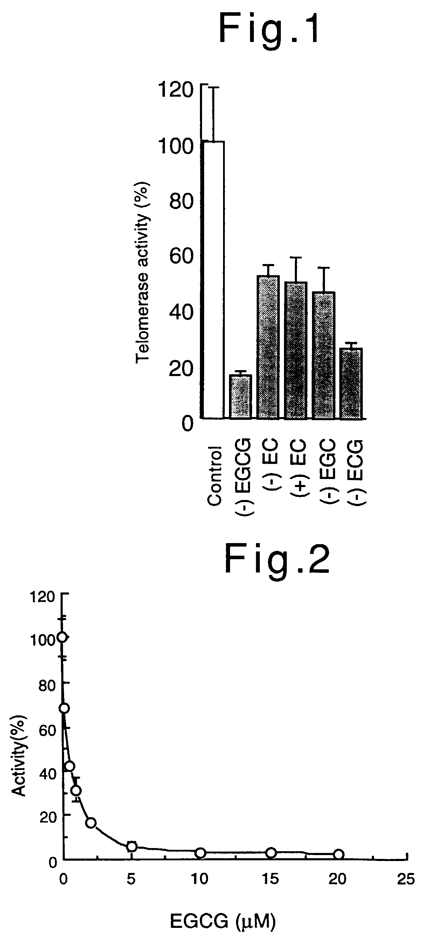

Results are shown in Figure 1. From Figure 1, it is evident that EGCG has the highest inhibiting activity among the five catechins.

### Example 2

### Telomerase inhibiting activity of EGCG

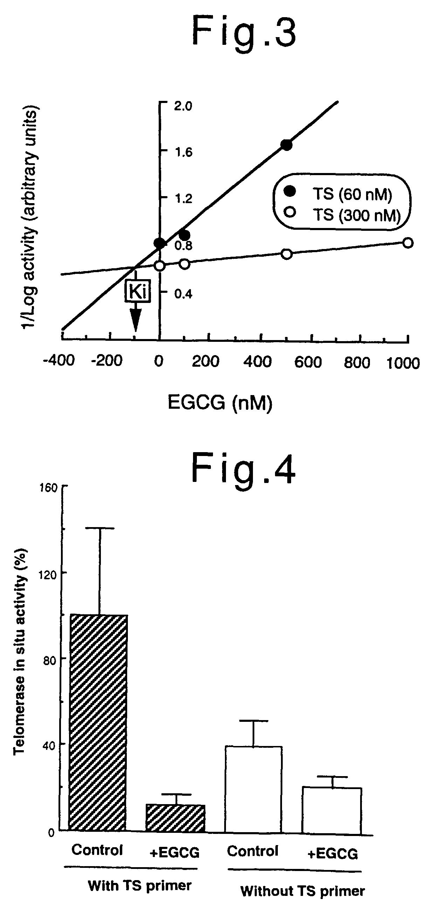

Telomerase activity in reaction solutions of 8 different EGCG concentrations ranging from 0.1 to 20 µM was measured in the same manner as described in Example 1 except that a primer, PS, was used at two different concentrations. Results are shown in Figure 2. A Dixon plot was plotted from the results in Figure 2, which gave a Ki value of about 100 nM (see Figures 2 and 3).

### Example 3

### Telomerase inhibiting activity of tea extract

Telomerase activity in reaction solutions containing powder sample 1 obtained in Production Example 1, powder sample 2 (a tea catechin extract: THEA-FLAN 90S, a trade name, a product of ITO EN, Ltd), and EGCG at different concentrations in the following ranges was measured in the same manner as described in Example 1.
- Powder sample 1: 0.30 to 1.00 µg/ml
- Powder sample 2: 0.10 to 0.30 µg/ml
- EGCG: 0. 10 to 0.30 µg/ml

The composition of THEA-FLAN 90S is as follows:
- Tea polyphenols: 92% by weight
- (-)-Epigallocatechin gallate: 50% by weight
- (-)-Epigallocatechin: 0% by weight
- (-)-Epicatechin gallate: 13% by weight
- (-)-Epicatechin: 0% by weight
- Caffein: 0.5% by weight

Inhibiting activity was evaluated by computing the concentration of a sample which suppresses the amplification level of DNA in a control by 50% (IC₅₀). Results are shown in Table 1.

**Table 1**

| Telomerase inhibition, IC₅₀. | |
|---|---|
| Sample | IC₅₀ |
| 1 | 0.88 |
| 2 | 0.24 |
| EGCG | 0.25 |

### Example 4

### Telomerase inhibiting activity in cells

The telomerase inhibiting activity of EGCG was assayed in monoblastoid leukemia U937 cells (American Type Culture Collection, ATCC) and colon adenocarcinoma HT29 cells.

An RPMI1640 medium (Nissui) supplemented with 10% fetal bovine serum (FBS) was used for U937 cells, and an RPMI1640 medium supplemented with 5% FBS and 5% fetal calf serum (FCS) was used for HT29 cells.

The cells were incubated in a 15 µM EGCG solution for 2 hours and washed in PBS. The washed cells were suspended in an RPMI1640 medium (without serum) at a concentration of 2 x 10⁶ cells/ml, then Streptolysin O (Sigma, 5 U/ml), TS primer (10 µM), spermidine (1 mM), and Imipramine (50 µM) were added, and the admixture was incubated at room temperature for 10 minutes. Streptolysin O is a substance which increases the permeability of a substance through the cell membrane, and enables the TS primers and other substances to be incorporated into the cell.

The permeabilization reaction was stopped by adding an equal volume of RPMI1640 medium supplemented with 10% FBS, after which the cell suspension was further incubated for 1 hour to seal the cell membrane. The cells thus obtained, in which TS primer and other substances were incorporated, were incubated at room temperature for 1 hour to coat the cell membrane, and at the same time, to allow the intracellular telomerase reaction to progress. The cells were then washed twice with PBS, pelleted, and frozen for a TRAP assay to detect telomerase products.

Namely, the cells were washed with ice-cold PBS and suspended again in PBS to count the cells. This suspension was centrifuged, the resulting cell mass was suspended in a TRAP assay buffer, then the suspension was quick-frozen with liquid nitrogen. After melting, the suspension was allowed to stand in an ice bath for 30 minutes, then the resulting extract was centrifuged to obtain the supernatant as a cell extract. TS primer (344 nM), ACX primer (385 nM), 50 µM dNTPs, T4g32 protein (Behringer-Manheim), an internal standard for PCR, TSNT (0.02 pM), and NT primer (385 nM) were added to a specified volume of the cell extract (corresponding to 200 to 1000 cells), then PCR was performed in the presence of Gene Taq DNA polymerase (2 U), i.e., (94°C, 40 seconds → 50°C, 40 seconds → 72°C, 60 seconds) x 35 cycles → 72°C, 2 minutes, to obtain PCR products.

Base sequences of the primers used in this reaction are as follows.

### ACX primer:

5'-GCGCGGCTTACCCTTACCCTTACCCTAACC-3' (SEQ ID NO: 4)

### TSNT:

5'-AATCCGTCGAGCAGAGTTAAAAGGCCGAGAAGCGAT-3' (SEQ ID: NO. 5)

### NT primer:

5'-ATCGCTTCTCGGCCTTTT-3' (SEQ ID NO: 6)

The telomerase products thus obtained were resolved by polyacrylamide gel electrophoresis, after which DNA bands were stained with SYBR Green (Takara Shuzo) and detected by irradiation with a UV transilluminator. The intensity of each band was quantitatively analyzed using the NIH Image 1.60 software program and telomerase activity was expressed as the intensity of a sample band relative to that of the band corresponding to the ITAS fragment (control).

In the abovementioned PCR, a part elongated by telomerase was amplified using TS primer as the forward primer and ACX primer as the return primer. Further, the 36-bp internal standard (TSNT) and its own return primer (NT) were used as described by Kim et al. (Kim, N.W. and Wu, F., Nucleic Acids Res., 25, 2595-2597, 1997).

Results are shown in Figure 4. The results showed that the treatment of the cells with EGCG markedly inhibited intracellular telomerase activity. Minor signals were also detected in the absence of TS primer. These signals were deemed to be derived from endogenous telomere sequences, or the like, and was similarly inhibited by the EGCG treatment.

### Example 5

### Mechanism of effect of EGCG on telomerase

The cells were treated with 15 µM EGCG for 2 hours and washed with ice-cold PBS. The cell mass obtained by centrifugation was suspended in a TRAP assay buffer and the resulting suspension was quick-frozen with liquid nitrogen. After melting, the suspension was allowed to stand in an ice bath for 30 minutes, then the resulting extract was centrifuged to obtain the supernatant as a cell extract. A test compound, TS primer (50 ng), 50 µM dNTPs, and T4g32 protein (Behringer-Manheim) were added to a specified volume of the cell extract (corresponding to 200 to 1000 cells), and the admixture was incubated at 20°C for 30 minutes. After reaction, CX primer (100 ng) was added to the reaction solution, then PCR was performed in the presence of Gene Taq DNA polymerase (2 U) and ITAS (10⁻¹⁸ g), i.e., (94°C, 40 seconds → 50°C, 40 seconds → 72°C, 60 seconds) x 30 cycles → 72°C, 2 minutes, to obtain PCR products. The PCR products thus obtained were resolved by polyacrylamide gel electrophoresis and the resulting DNA bands stained with SYBR Green (Takara Shuzo) were detected by irradiation with a UV transilluminator. The intensity of each band was quantitatively analyzed using the NIH Image 1.60 software program. Telomerase activity was expressed as the intensity of a sample band relative to that of the band corresponding to the ITAS fragment.

The abovementioned procedure was carried out for both U937 cells and HT29 cells. Results showed that the EGCG treatment of cells had no effect on telomerase activity, when tested in vitro because of the dilution effect during the solubilization of cells. This observation along with the results of Example 1, 2, and 4 revealed that EGCG directly and reversibly inhibited telomerase activity in the cells, and that this inhibition was not through the intracellular information transfer system (intracellular signaling pathway).

### Example 6

U937 cells and HT29 cells were each subcultured on a medium with and without EGCG to study the effect of addition of EGCG on cell growth. The medium used was the same as used in Example 4.

Five subculture lines for U937 cells and nine subculture lines for HT29 cells were prepared. The first culture of each line was made by inoculating cells of each stock culture at a density of 5 x 10⁵ cells per 10-cm dish. For each subculture, two lines were cultured on a medium without EGCG (control lines) and the rest of the lines were cultured on a medium supplemented with 15 µM EGCG.

Cells were passaged, sampled and counted every four days. The incubation medium was freshly changed every day.

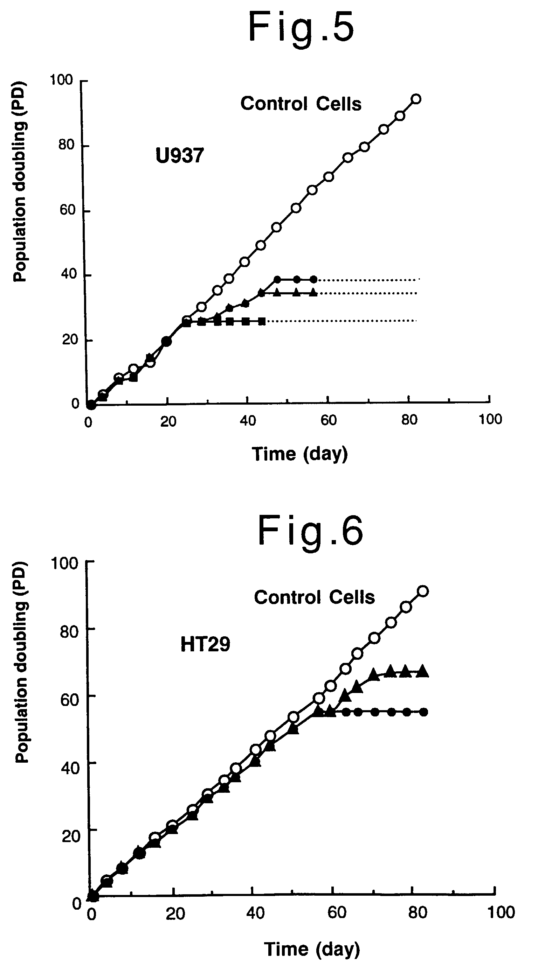

Results are shown in Figure 5 (U937 cells) and Figure 6 (HT29 cells). Symbol ○ shows the control line cultured on a medium without EGCG. In Figure 5, symbols ●, ▲, and ■ show the lines cultured on a medium supplemented with 15 µM EGCG. Cells of all the lines entered cell crisis at day 53 (●), day 44 (▲), and day 29 (■), and thereafter showed morphological changes similar to cell senescence. In Figure 6, both symbols ▲ and ● show the lines cultured on a medium supplemented with 15 µM EGCG. Cells of both lines entered cell crisis at day 75 (▲) and day 57 (•), and thereafter showed molphological changes similar to cell senescence.

U937 cells and HT29 cells entered crisis approximately at PD25 and PD60, respectively, showing characteristic morphological changes (rounding up and detachment for HT29 cells and raggedness of the plasma membrane for U937 cells).

U937 cells died at PD40 and HT 29 cells died at PD70. PD is the number of cycles in which the cell population doubles during cell growth and is calculated by monitoring cell counts using a hemocytometer.

The form of the cells did not revert and was maintained when the cells with morphological changes were transferred and cultured on a medium without EGCG, which confirmed that the morphological changes were not temporary.

HT 29 cells from PD57 to PD60 in cell crisis were pooled and the genomic DNA was prepared by a salting-out/ethanol precipitation method (Stratagene). Further, integrity of the isolated genomic DNA was confirmed by gel electrophoresis (ethidium bromide staining).

The isolated genomic DNA (10 µg) was digested with HinfI and RsaI and the resulting digests were subjected to Southern blotting by the ordinary method, after which DNA fragments were detected using a TeloQuant assay kit (PharmMingen).

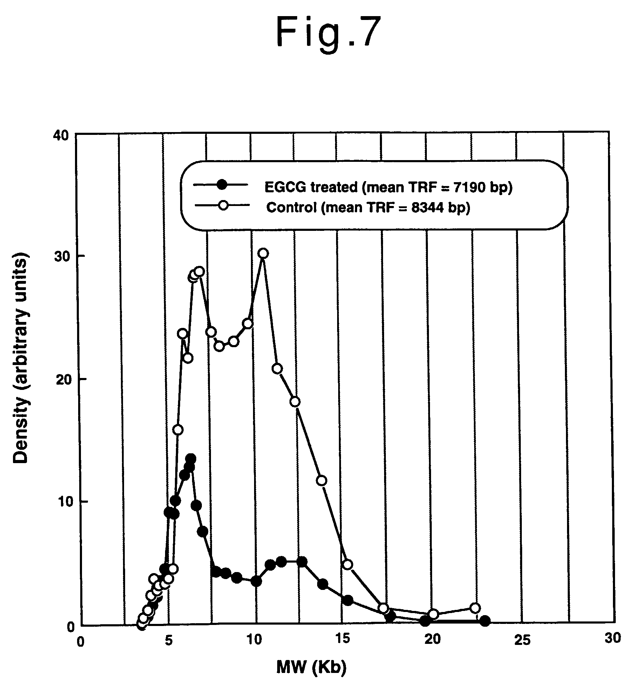

Results are shown in Figure 7. While cleavage sites recognized by restriction enzymes HinfI and RsaI are numerous in a normal genomic DNA, such cleavage sites do not exist in the subtelomere or telomere region. Accordingly, if a genoinic DNA is digested with the abovementioned restriction enzymes, only the telomere region would remain undigested. This DNA fragment is called the Terminal Restriction Fragment (TRF) which can be detected by Southern blotting to analyze telomere lengths. TRFs of EGCG-treated or untreated HT29 cells obtained as mentioned above were detected using a TeloQuant assay kit. Figure 7 shows the results of densitometric analysis of the detected signals. It was observed that TRFs in the cells which entered crisis due to the EGCG treatment (•) were shortened by about 1.1 kb as compared to those in the untreated cells( 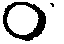 ). Further, it was confirmed that successive EGCG treatment of HT29 cells caused telomere diminishment.

Further, analysis of DNA content of cells before entering crisis by the ordinary method using flowcytometry revealed a 50 to 100% and a 10 to 20% increase in the aneuploidy and cell cycle G2/M fractions, respectively, for both U937 cells and HT29 cells.

In conclusion, it is evident from these results that cell crisis to death was caused by intracellular telomerase inhibition by EGCG.

SEQUENCE LISTING

SEQ ID NO: 1
SEQUENCE TYPE: Nucleotide
SEQUENCE LENGTH: 18 bases
STRANDNESS: Single
TOPOLOGY: Linear
MOLECULE TYPE: Synthetic DNA
PROPERTIES: TS primer for PCR
SEQUENCE:
AATCCGTCGA GCAGAGTT   18

SEQ ID NO: 2
SEQUENCE TYPE: Nucleotide
SEQUENCE LENGTH: 28 bases
STRANDNESS: Single
TOPOLOGY: Linear
MOLECULE TYPE: Synthetic DNA
PROPERTIES: CX primer for PCR
SEQUENCE:
GTGCCCTTAA CCCTTACCCT TACCCTAA

SEQ ID NO: 3
SEQUENCE TYPE: Nucleotide
SEQUENCE LENGTH: 150 bases
STRANDNESS: Single
TOPOLOGY: Linear
MOLECULE TYPE: Synthetic DNA
PROPERTIES: ITAS internal standard for PCR

SEQUENCE:

AATCCGTCGA GCAGAGTTGT GAATGAGGCC TTCGAGGCTC 40

TGAAGAGAAG CACCCTGCTC AACCCCAACC AGCGGCTGCC 80

TAAGGTGGAG ATCCTGCGCA GTGCCATCCA GTACATTGAG 120

CGCCTATTAG GGTAAGGGTA AGGGTAAGGG 150

SEQ ID NO: 4

SEQUENCE TYPE: Nucleotide

SEQUENCE LENGTH: 30 bases

STRANDNESS: Single

TOPOLOGY: Linear

MOLECULE TYPE: Synthetic DNA

PROPERTIES: ACX primer for PCR

SEQUENCE:

GCGCGGCTTA CCCTTACCCT TACCCTAACC 30

SEQ ID NO: 5

SEQUENCE TYPE: Nucleotide

SEQUENCE LENGTH: 36 bases

STRANDNESS: Single

TOPOLOGY: Linear

MOLECULE TYPE: Synthetic DNA

PROPERTIES: TSNT internal standard for PCR

SEQUENCE:

AATCCGTCGA GCAGAGTTAA AAGGCCGAGA AGCGAT

SEQ ID NO: 6

SEQUENCE TYPE: Nucleotide

SEQUENCE LENGTH: 18 bases

STRANDNESS: Single
TOPOLOGY: Linear
MOLECULE TYPE: Synthetic DNA
PROPERTIES: NT primer for PCR
SEQUENCE:
ATCGCTTCTC GGCCTTTT 18

## Claims

1. A telomerase inhibitor comprising a catechin as an effective component.

2. A telomerase inhibitor according to claim 1 comprising a green tea extract containing a catechin or a mixture of catechins.

3. A telomerase inhibitor according to claim 1 or 2 wherein the catechin is epigallocatechin gallate, epigallocatechin, epicatechin gallate, or epicatechin.

4. A telomerase inhibitor according to claim 1, 2, or 3 further comprising a pharmaceutically acceptable carrier or diluent.

5. A telomerase inhibitor according to any of claims 1 to 4 wherein the catechin content is 90 to 95% by weight.

6. A cancer prevention or anticancer agent comprising a catechin as an effective component.

7. A cancer prevention or anticancer agent according to claim 6 comprising a green tea extract containing a catechin or a mixture of catechins

8. A cancer preventing or anticancer agent according to claim 6 or 7 wherein the catechin is epigallocatechin gallate, epigallocatechin, epicatechin gallate, or epicatechin.

9. A cancer prevention or anticancer agent according to claim 6, 7, or 8 further comprising a pharmaceutically acceptable carrier or diluent.

10. A cancer prevention or anticancer agent according to any of claims 6 to 9 wherein the catechin content is 90 to 95% by weight.

11. A method of inhibiting a telomerase comprising contacting the telomerase with a telomerase inhibitor as claimed in any one of claims 1 to 5.

12. Use of a catechin in manufacture of a medicament for preventing or treating cancer.

13. Use of a catechin for production of a telomerase inhibitor.

14. Use of a catechin for production of a cancer prevention or anticancer agent.

15. A method of producing a telomerase inhibitor comprising a step of formulation of the telomerase inhibitor using a catechin as an effective ingredient.

16. A method of producing a cancer prevention or anticancer agent comprising a step of formulation of the cancer prevention or anticancer agent using a catechin as an effective ingredient.

17. A catechin for use as a telomerase inhibitor.

18. A catechin for prevention or treatment of cancer.
